# EUROPEAN PATENT APPLICATION

(11) **EP 0 531 027 A1**
(43) Date of publication of application: **10.03.1993**
(21) Application number: 92307660.8
(22) Date of filing: 21.08.1992
(51) Int. Cl.: C12Q 1/68, C07H 21/00, C12P 19/34

(54) **Labelled primers for nucleic acid probes**

(30) Priority: 03.09.1991 GB 9118785
(71) Applicant: AMERSHAM INTERNATIONAL plc, Little Chalfont Buckinghamshire HP7 9NA (GB)
(72) Inventor: Evans, Michael Robert, Hazlemere, Buckinghamshire HP15 7AG (GB); Craig, Frank Furlong, Wendover, Buckinghamshire HP22 6PF (GB)
(74) Representative: Pennant, Pyers

(57) **Abstract**

A method of making labelled probes for a target nucleic acid sequence is described. The target sequence is contacted with a plurality of nucleic acid primers under hybridising and chain extension conditions. The primers used in the method are multiply labelled at their 5'-ends. Also described is a kit for making labelled probes comprising a random mixture of oligonucleotide primers in which each primer is multiply labelled at its 5'-end.

## Description

This invention relates to labelled DNA primers and their use in producing nucleic acid probes. It relates to improvements in the labelling of DNA primers and in sensitivity of detection of aforesaid primers after their use for the generation of nucleic acid probes. The main use for such probes is for detection of specific target sequences of nucleic acid in hybridisation techniques. The probes can be made in better yields and have improved hybridisation efficiency and sensitivity.

### Brief Description of the Prior Art

Nucleic acid hybridisation is a powerful technique for detection of specific, complementary nucleic acid sequences and can be used for detection of viruses, bacteria and genetic disorders.

Hybridisation involves the use of nucleic acid probes which are complementary in sequence to the target nucleic acid. The hybrids may then be detected using assays for the probes which are either measured directly or indirectly (Mathews, J. A. & Kricka, L. J. (1988). Anal. Biochem. 169, 1-25). Direct labels include phosphorus-32, fluorescein, horseradish peroxidase and alkaline phosphatase. The simplest indirect procedure involves uses of a specific antibody which recognises a DNA:DNA or DNA:RNA duplex. A variant of the indirect procedure involves a DNA probe labelled with a hapten. The duplex:antibody complex or duplex:hapten is then detected using a labelled second antibody (Mathews, J. A. & Kricka, L. J. (1988). Anal. Biochem. 169, 1-25). Biotin is one of the most often used haptens in indirect labelling procedures and is normally detected with a labelled anti-biotin antibody (Langer, P. R. et al. (1981). Proc. Natl. Acad. Sci. USA 78, 6633-6637) or a labelled avidin or streptavidin complex (Pollard-Knight, D. et al. (1990). Anal. Biochem. 185, 353-358). Another possible technique of detecting hybridisation is by the use of probes followed by the addition of agents which can intercalate into double-stranded nucleic acid. Each intercalating molecule could have a linked label which would then allow detection of hybrids (Levenson, C. H. & Mullis, K. B. (1988). United States Patent No. 4,751,313).

The use of random sequence primers permits labelling of DNA from any source without the need for specific vectors or for sub-cloning. The "Klenow" fragment of DNA polymerase I is used for this reaction. The absence of the 5'-3' exonuclease activity normally associated with DNA polymerse I ensures that nucleotides incorporated by the polymerase are not subsequently removed. The usefulness of the random priming approach to the generation of labelled probes has led to the commercial supply of random priming kits, such as the "Multiprime" kit sold by Amersham International plc. The technique has been described, (Feinberg, A. P. & Vogelstein, B. (1983). Anal. Biochem. 132, 6-13).

In the primer extension technique, labels, haptens or functional moieties are currently attached to the probe in one of three ways. The first technique is by direct incorporation of the hapten into the newly synthesised complementary sequence using labelled nucleotides such as biotin-11-dUTP. The second technique involves incorporation of modified nucleotides with functional moieties which are reacted later with reporter groups to give a labelled product. One example is the use of modified nucleotides with alkylamine linker-arms to which reporter groups can later be linked using their N-hydroxy succinimide esters (Urdea, M. S. et al. (1988). Nucleic Acids Res. 16, 4937-4956; Arnold, L. J., Jr. et al. (1989). Clin. Chem. 35, 1588-1594). A noticeable similarity with these two labelling techniques is that only a single hapten or reporter group can be attached to each modified nucleotide. The last technique involves direct labelling of the probe by treatment of the probe with a reporter enzyme under conditions which allow direct cross-linking of the probe and the enzyme to give a labelled product. This technique has been described for horseradish peroxidase (Renz, M. & Kurz, C. (1984). Nucleic Acids Res. 12, 3435-3444) and was later used by Amersham International plc to give a range of commercial molecular biology kits (Durrant, I. et al. (1990). Biotechniques 8, 564-570). This technique appears to give one enzyme molecule per every 50 bases.

A recent International Patent Application WO 91/17169 (Amersham) describes the generation of novel phosphoramidite derivatives, particularly biotinyl and phosphotyrosinyl phosphoramidite compounds. The inventors proposed that these would be useful for the incorporation of single or multiple reporter groups at the 5' end of synthetic oligonucleotides. In addition, other workers have described the incorporation of up to 10 biotin residues on the 3'-end of an oligonucleotide and its subsequent use as a probe (Haralambidis, J. et al. (1990). Nucl. Acids. Res. 18, 501-505). Neither report suggests the use of these oligonucleotides as labelled primers which can then be used for production of long, nucleic acid probes.

### Summary of Invention

In one aspect the invention provides a method of making labelled probes for a target nucleic acid sequence, which method comprises contacting the target sequence with a plurality of nucleic acid primers under hybridising and chain extension conditions, characterised in that the primers are multiply labelled at their 5'-ends.

In another aspect the invention provides a kit for making labelled probes comprising a random mixture of oligonucleotide primers in which each primer is multiply labelled at its 5'-end.

The invention uses nucleic acid primers which are labelled at the 5'-end with more than one label to produce probes by primer extension. Since a tail of labels on the primer are joined onto a single 5' modified nucleotide, this approach has various benefits over using probes with either a single 5' label or with an unlabelled primer with multiple, internal labels (one attached to each modified nucleotide) incorporated during primer extension. These benefits include:
1) Little or no interference of the extra labels with hybridisation of the probe to its target nucleic acid sequence.
2) An increase in probe yield since modified nucleotides, such as biotin-11-dUTP, do not have to be used for incorporation during primer extension.
3) A greater affinity of hybridisation than a probe with many single, modified nucleotides.
4) Increased sensitivity of detection over probes with a single 5'-label and possibly over probes containing numerous, incorporated internal labels.

### Description of Invention

The method of making labelled probes for a target nucleic acid sequence involves contacting the target sequence with a plurality of nucleic acid primers. Under the hybridising conditions used, some at least of those primers become hybridised to the target sequence. In the presence of a polymerase enzyme and a supply of nucleotide triphosphates, the primers initiate chain extension resulting in labelled probes complementary to the target sequence, which are subsequently recovered by standard techniques.

Although not necessary, it is often convenient to use a random mixture of oligonucleotide primers, generally 5 to 20 and preferably 6 to 9 nucleotides in length. Some of these primers hybridise to the target sequence. The lengths of the primers and the size of the random primer mix together determine the average spacing of hybridisation events along the length of the target nucleic acid sequence. This average spacing may be chosen to be a few hundred nucleotides, in order to obtain labelled probes whose average length is a few hundred nucleotides. Other variables which can be used to control the average length of the labelled probes include the choice of polymerase enzyme, the supply of nucleotide triphosphates and the incubation conditions. In particular, one or more of the nucleotide triphosphates can itself be labelled, but this is generally not necessary as the primers are already labelled at their 5'-ends, and tends to reduce the efficiency of probe production and is not preferred.

Alternatively, when the nucleotide sequence of the target is known, labelled primers of known sequence corresponding to the target may be chosen to hybridise to the target at desired intervals along its length.

The primers used in this invention are multiple labelled, that is to say they contain at least two labelled groups per primer molecule. These two (or more) labelled groups are generally the same, but may be different, and there may be positive advantages in providing primers having two or more different labelled groups attached to the 5'-end of each molecule. There is no critical upper limit on the number of labelled groups per primer molecule. Primers with more label groups are more easily and sensitively detected. The examples below show primers containing 2 to 8 labelled groups per molecule, but larger numbers up to 50 or even more are expected to show benefits.

The labelled groups may comprise haptens or other detectable moieties. Examples include biotin, dinitrophenyl, fluorescein and other related fluorescing moieties, phosphotyrosine, and dansyl. These label groups may be introduced at the 5'-ends of the primers by phosphoramidite chemistry, for example as described in the aforesaid International Patent Application WO 91/17169.

In the resulting probes, the label groups may be detected by a variety of known methods including antibody detection techniques, the fluorescent or colourigenic or chemiluminescent or bioluminescent property of the label group, or by the ability of the labelled group to act as a substrate or enhancer for a detectable enzymatic or chemical reaction. A kit for making labelled probes may comprise the random mixture of labelled oligonucleotide primers, preferably together with one or more of the following:
- a polymerase enzyme,
- a supply of nucleotide triphosphates for incorporation in the probes,
- a supply of reaction buffer,
- a supply of vessels for performing the hybridisation and chain extension reactions and for recovering the labelled probes from the target sequence.

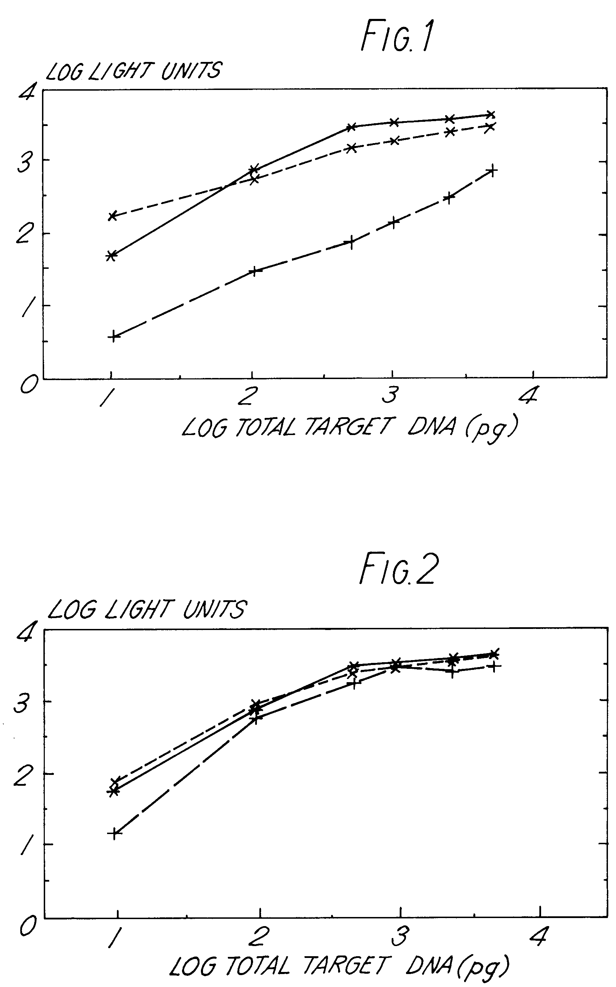

Reference is directed to Figures 1 and 2 of the accompanying drawings, both of which are graphs of log light units against log total target DNA. These figures are referred to in Examples 3 and 5 below.

In the following experimental section, Example 1 is a description of a method of making and using labelled DNA probes. Examples 2 to 5 are comparative experiments which demonstrate particular advantages.

### Example 1

### Generation of random oligonucleotide primers which are multiple-labelled at their 5'-end

Random primers (9-mers) were synthesised on an Applied Biosystems 380-B DNA synthesiser. Four syntheses were performed, representing the four nucleotides (A, T, G and C) at the 5' end with random 8-mers thereafter. Three sets were synthesised with additional differences at the 5'-end of the sequence: unlabelled or labelled with one biotin or a tail of 8 biotins. All synthesis reagents were supplied by Applied Biosystems except Biotinyl phosphoramidite compound which was supplied by Dr. M. J. Gait (MRC Laboratory of Molecular Biology, Hills Rd, Cambridge). After assembly the oligonucleotides were heated to 55°C in a sealed tube overnight and then evaporated to dryness. The residue was dissolved in 0.3 ml acetic acid/water (8:2 (v/v)) and, after 20 min at room temperature, the mixture was evaporated to dryness. To the residue, 0.5 ml of sterile, distilled water was then added and the suspension filtered through a 0.45 µm membrane filter.

### Purification of random, oligonucleotide primers

Reverse-phase high performance liquid chromatography was performed on a Beckman Ultrasphere column using gradients of buffer A (0.1 M ammonium acetate) and buffer B (20% (v/v)) buffer A (80% (v/v) acetonitrile) at a flow-rate of 1.0 ml min⁻¹. The major peak of each run was collected and evaporated to dryness.

### Production of probes by primer extension

Each primer suspension was calibrated to an E₂₆₀ of 15.00. A random primer mix was then made by mixing each of the respective suspensions (nucleotides A, T, G and C at the 5'end) to produce 3 random primer suspensions which were unlabelled or labelled at the 5'-end with either 1 or 8 biotins. An aliquot (10 µl) of each was then used to produce probes in a standard 50 µl Multiprime reaction according to manufacturer's instructions (Amersham International plc, RPN 1600). The target DNA used was lambda (Amersham, T 3010, 100 ng per tube) and the Klenow enzyme concentration was increased to 2 units ml⁻¹ per reaction (Amersham T 2141Y). To produce internally labelled probes, dTTP in the reaction mixture was replaced with biotin-11-dUTP. The concentration of probe produced was estimated by figures obtained from incorporation of ³²P-dCTP under non-limiting conditions.

### Use of probes in hybridisations

Dot-blots of lambda target DNA were fixed by baking at 80°C for 2 h under reduced atmospheric pressure. Hybond-ECL membranes were used and the hybridisations performed for nitrocellulose membranes as suggested by the manufacturer (Amersham, RPN 82D). The probe concentration used was 20 ng ml⁻¹. After hybridisation, the membrane was first blocked with 5% (w/v) reconstituted milk powder in tris buffered saline containing 0.1% Tween 20 (TBST) for 1 h. The membrane was then incubated for 45 min with a mouse monoclonal antibody against biotin (0.1 µg ml⁻¹ in TBST) and then washed with TBST (6x5 min). A sheep anti-mouse IgG-horseradish peroxidase conjugate was then added (Amersham, RPN 2020D) at a 1 in 1000 dilution in TBST. After incubation at room temperature for 45 min, the membranes were then washed with TBST (6x5 min) and finally tris buffered saline (TBS) (1x5 min). Detection was carried out using the enhanced chemiluminescence detection reagents (Amersham, RPN 2105). Luminescence from the dot-blots was quantitated using 300 sec exposures in a liquid nitrogen-cooled, charge-coupled device camera (Wright Instruments, Enfield, Middlesex, UK). The camera software calculates the luminescence over each specific dot and then expresses the mean light output per pixel.

### Example 2

### Priming efficiency of random primers which were multiple-labelled at the 5'-end

The random primers (unlabelled or labelled with 1 or 8-biotin at the 5'-end) were used to generate probes against lambda target DNA. The concentration of probe generated was estimated using incorporation of ³²P-dCTP under non-limiting conditions. With the unlabelled primer, a biotin label was incorporated in the extended nucleic acid sequence by the replacement of dTTP with biotin-11-dUTP (Table 1). The results show that even with the presence of 8 biotins on the primer the yield of probe generated was still higher (about 40%) than that generated by the unlabelled primer. This suggests that primers with multiple labels at the 5'-end do have an advantage as unmodified nucleotides can be used for primer extension. These are known to be incorporated at a faster rate than modified nucleotides which have to be used with unlabelled primers.

**Table 1**

| Yield of probe generated by each primer as calculated from ³²P-dCTP incorporation | | | |
|---|---|---|---|
| Primer | Biotin-11-dUTP incorporated | % ³²P-dCTP incorporation* | Yield of probe (ng) |
| Unlabelled | + | 5.4 | 91 |
| 1-biotin | - | 16.1 | 271 |
| 8-biotin | - | 7.6 | 128 |

| | | | |
|---|---|---|---|
| * Mean of two experiments | | | |

### Example 3

### Detectability of multiple-labelled, random primers and associated probe sequences

The probes produced were assayed in hybridisations for sensitivity of detection of dot-blots of lambda target DNA (Figure 1). All primers were detectable and the 8-biotin labelled primer was more easily detectable than the primer labelled with only one biotin. This suggested that a correlation exists between length of 5' biotin tail and ease of detection. The 8-biotin tailed probe and the probe prepared with the unlabelled primer both detected down to 10 pg of target DNA whereas the 1-biotin tailed probe only detected 100 pg of target DNA. Possibly by further increasing the length of the biotin tail the sensitivity of detection may be increased such that it is superior to that obtained using the probe generated from the unlabelled primer.
Figure 1: Detection of target DNA using probes generated by the different primers. Results shown are corrected for background and are a mean of 3 separate observations. Only values above a log light unit value of about 1.30 (i.e. twice background) were taken as significant. In Figure 1:
- Unlabelled primer is shown by a solid line.
- 1-Biotin primer is shown by a dashed line.
- 8-Biotin primer is shown by a dotted line.

### Example 4

### Incorporation of internal labels into probes generated from primers which are multiple-labelled at their 5'-end

The multiple-labelled primers were investigated to assess their ability to incorporate a labelled nucleotide (biotin-11-dUTP) during probe production by primer extension. Using similar conditions as above (Table 1), it was found that the multiple-labelled primers could incorporate biotin-11-dUTP at approximately the same efficiency as an unlabelled primer (Table 2).

**Table 2**

| Effect of incorporation of biotin-11-dUTP on probe yield generated by each primer as calculated from ³²P-dCTP incorporation | | |
|---|---|---|
| Primer | % ³²P-dCTP incorporation* | Yield of probe (ng) |
| Unlabelled | 5.4 | 91 |
| 1-biotin | 5.2 | 87 |
| 8-biotin | 4.2 | 70 |

| | | |
|---|---|---|
| * Mean of two experiments | | |

### Example 5

### Detectability of internal labels by hybridisation

The probes with additional, internal labels (biotin-11-dUTP) were assayed for their detectability after hybridisation against their target DNA. All probes were detectable (Figure 2) and the sensitivity of detection of the 1-biotin labelled primer with internal biotin labels appeared to be greater than that achieved when no internal labels were incorporated (Figure 1). The internally labelled probe made from the 8-biotin labelled primer apparently showed no more sensitivity than that obtained when no labels were incorporated.
Figure 2: Detection of target DNA using internally labelled probes generated by the different primers. Results shown are corrected for background and are a mean of 3 separate observations. Only values above a log light unit value of about 1.30 (i.e. twice background) were taken as significant. In Figure 2:
- Unlabelled primer is shown by a solid line.
- 1-Biotin primer is shown by a dashed line.
- 8-Biotin primer is shown by a dotted line.

## Claims

1. A method of making labelled probes for a target nucleic acid sequence, which method comprises contacting the target sequence with a plurality of nucleic acid primers under hybridising and chain extension conditions, characterised in that the primers are multiply labelled at their 5'-ends.

2. A method as claimed in Claim 1, wherein a random mixture of primers is used.

3. A method as claimed in Claim 1 or Claim 2, wherein the primers are oligonucleotide primers 6 to 9 nucleotides in length.

4. A method as claimed in any one of Claims 1 to 3, wherein the primers contain at least one label selected from biotin, fluorescein and phosphotyrosine.

5. A method as claimed in any one of Claims 1 to 4, wherein the 5'-labels have been incorporated in the primers using a phosphoramidite.

6. A kit for making labelled probes comprising a random mixture of oligonucleotide primers in which each primer is multiply labelled at its 5'-end.

7. A kit as claimed in Claim 6, comprising also:
- a polymerase enzyme,
- a supply of nucleotide triphosphates for incorporation in the probes.

8. A kit as claimed in Claim 6 or Claim 7, wherein the primers contain at least one label selected from biotin, fluorescein and phosphotyrosine.

9. A kit as claimed in any one of Claims 6 to 8, wherein the 5'-labels have been incorporated in the primers using a phosphoramidite.
